(19) Europäisches Patentamt
European Patent Office
Office européen des brevets

(11) Veröffentlichungsnummer: **0 413 226 B1**

(12) **EUROPÄISCHE PATENTSCHRIFT**

(45) Veröffentlichungstag der Patentschrift: **24.05.95**

(51) Int. Cl.6: **C07D 471/04**, //(C07D471/04, 249:00,221:00)

(21) Anmeldenummer: **90115072.2**

(22) Anmeldetag: **06.08.90**

# (54) [1,2,4] Triazolo [1,5-a]pyridine.

(30) Priorität: **12.08.89 DE 3926770**

(43) Veröffentlichungstag der Anmeldung:
**20.02.91 Patentblatt 91/08**

(45) Bekanntmachung des Hinweises auf die Patenterteilung:
**24.05.95 Patentblatt 95/21**

(84) Benannte Vertragsstaaten:
**CH DE FR GB IT LI**

(56) Entgegenhaltungen:
**FR-A- 2 351 978**

**SYNTHESIS, Nr. 10, Oktober 1986, Seiten 860-862, Stuttgart, DE; D.W. RANGNEKARet al.: "A novel, one-step synthesis of [1,2,4] triazolo[1,5-a]-pyridinederivatives"**

(73) Patentinhaber: **BASF Aktiengesellschaft**
**Carl-Bosch-Strasse 38**
**D-67063 Ludwigshafen (DE)**

(72) Erfinder: **Schefczik, Ernst, Dr.**
**Dubliner Strasse 7**
**D-6700 Ludwigshafen (DE)**
Erfinder: **Reichelt, Helmut, Dr.**
**Johann-Gottlieb-Fichte-Strasse 56**
**D-6730 Neustadt (DE)**

EP 0 413 226 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die vorliegende Erfindung betrifft neue Triazolopyridine der Formel I

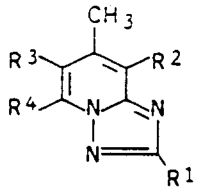

(I),

in der

$R^1$ $C_1$-$_{20}$-Alkyl, das gegebenenfalls durch Phenyl, Phenoxy, Carboxyl oder $C_1$-$C_{20}$-Alkoxycarbonyl, dessen Alkylkette gegebenenfalls durch 1 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alkyl, $C_1$-$C_4$-Alkoxy, Halogen, Nitro oder Carboxyl substituiertes Phenyl oder Hydroxy,

$R^2$ Wasserstoff, Formyl, Nitroso, Cyano, $C_1$-$C_4$-Alkoxymethyl oder einen Rest der Formel $CH_2$-$NL^1L^2$, wobei $L^1$ und $L^2$ gleich oder verschieden sind und unabhängig voneinander jeweils Wasserstoff oder zusammen mit dem sie verbindenden Stickstoffatom einen 5- oder 6-gliedrigen gesättigten heterocyclischen Rest,

$R^3$ Wasserstoff, Cyano, Carbamoyl, Carboxyl oder $C_1$-$C_4$-Alkoxycarbonyl und

$R^4$ Hydroxy, Halogen, den Rest -$NL^1L^2$, wobei $L^1$ und $L^2$ jeweils die obengennante Bedeutung besitzen, oder den Rest einer CH-aciden Verbindung bedeuten, die sich von Nitromethan, Nitroethan oder von Verbindungen der Formeln V bis X

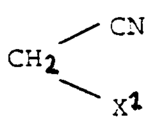

(V)

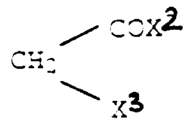

(VI)

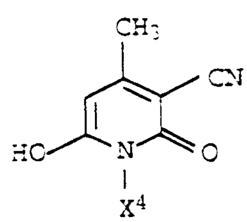

(VII)

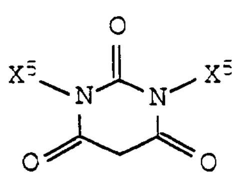

(VIII)

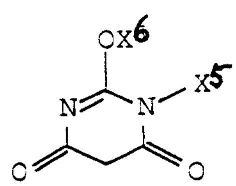

(IX)

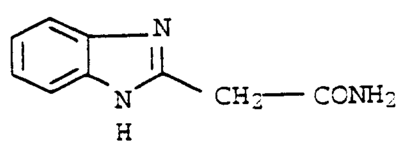

(X)

ableitet, wobei

$X^1$ Cyano, Nitro, $C_1$-$C_4$-Alkanoyl, gegebenenfalls durch $C_1$-$C_4$-Alkyl, $C_1$-$C_4$-Alkoxy oder Halogen substituiertes Benzoyl, $C_1$-$C_6$-Alkylsulfonyl, gegebenenfalls durch $C_1$-$C_4$-Alkyl, $C_1$-$C_4$-Alkoxy oder Halogen substituiertes Phenylsulfonyl, Carboxyl, $C_1$-$C_4$-Alkoxycarbonyl, Phenoxycarbonyl, Carbamoyl, $C_1$-$C_4$-Mono- oder Dialkylcarbamoyl, gegebenenfalls durch $C_1$-$C_4$-Alkyl, $C_1$-$C_4$-Alkoxy oder Halogen substituiertes Phenylcarbamoyl, gegebenenfalls durch $C_1$-$C_4$-Alkyl, $C_1$-$C_4$-Alkoxy, Halogen oder Nitro substituiertes Phenyl, Benzthiazol-2-yl, Benzimidazol-2-yl, 5-Phenyl-1,3,4-thiadiazol-2-yl oder 2-Hydroychinoxalin-3-yl,

$X^2$ $C_1$-$C_4$-Alkyl oder $C_1$-$C_4$-Alkoxy,
$X^3$ $C_1$-$C_4$-Alkoxycarbonyl, Phenylcarbamoyl oder Benzimidazol-2-yl,
$X^4$ Wasserstoff oder $C_1$-$C_6$-Alkyl,
$X^5$ Wasserstoff, $C_1$-$C_4$-Alkyl oder Phenyl und
$X^5$ $C_1$-$C_4$-Alkyl bedeuten,

sowie ein Verfahren zur Herstellung von in 2-Stellung substituierten 5-Methyl-4- oder 6-cyano-7-hydroxy-[1,2,4]triazolo[1,5-a]pyridinen.

Aus Synthesis 1986, Seiten 860 bis 862, sind 4-Cyano-5,7-dimethyl[1,2,4]triazolo[1,5-a]pyridine bekannt, die in Ringposition 2 unsubstituiert oder durch Methyl, Phenyl, Styryl oder Cumarin-3-yl substituiert sind. Es hat sich jedoch gezeigt, daß diese Triazolopyridine für Folgereaktionen wenig geeignet sind, da sie, abgesehen von der Cyanogruppe, über keine weiteren funktionellen Gruppen am Pyridinring verfügen.

Aufgabe der vorliegenden Erfindung war es deshalb, neue Triazolopyridine bereitzustellen, die zusätzliche funktionelle Gruppen am Pyridinring aufweisen und die in einfacher Weise herzustellen sind.

Demgemäß wurden die eingangs näher bezeichneten Triazolopyridine der Formel I gefunden.

Die exakte IUPAC-Bezeichnung des Grundkörpers, dem die neuen Triazolopyridine der Formel I zugrundeliegen, lautet [1,2,4]Triazolo[1,5-a]pyridin, wobei die folgende Bezifferung der Ringe gilt

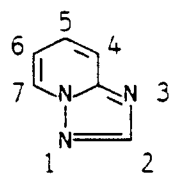

Die Verbindungen der Formel I können in mehreren tautomeren Formen auftreten, die alle vom Patentanspruch umfaßt werden. Beispielsweise können die Verbindungen mit $R^4$ = Hydroxy u.a. in folgenden tautomeren Formen auftreten:

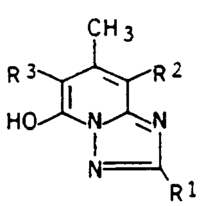

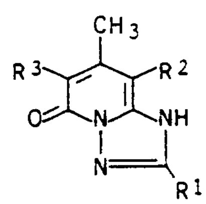

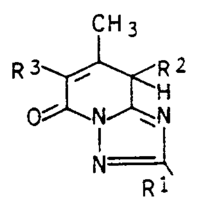

Wenn $R^1$ in Formel I einen Alkylrest darstellt, der durch Sauerstoffatome unterbrochen ist, so sind solche Alkylreste bevorzugt, die durch 1 bis 2 Sauerstoffatome unterbrochen sind.

Wenn $R^1$ in Formel I einen durch Halogen substituierten Phenylrest bedeutet, so können als Substituenten insbesondere Chlor oder Brom in Betracht kommen.

Als CH-acide Verbindungen sind insbesondere solche der Formel V, VI und VIII zu nennen, wobei

$X^1$ Cyano, Acetyl, Benzoyl, $C_1$-$C_4$-Alkoxycarbonyl, Phenoxycarbonyl, $C_1$-$C_2$-Monoalkylcarbonyl, Phenylcarbamoyl, Phenyl, Benzimidazol-2-yl, Benzthiazol-2-yl oder 5-Phenyl-1,3,4-thiadiazol-2-yl,
$X^2$ $C_1$-$C_2$-Alkoxy,
$X^3$ $C_1$-$C_2$-Alkoxycarbonyl oder Phenylcarbamoyl und
$X^5$ Methyl bedeuten.

Reste $R^1$ sind z. B. Wasserstoff, Methyl, Ethyl, Propyl, Isopropyl, Butyl, Isobutyl, sec-Butyl, Pentyl, Isopentyl, Neopentyl, tert-Pentyl, Hexyl, Heptyl, 1-Ethylpentyl, Octyl, Isooctyl, 2-Ethylhexyl, Nonyl, Isononyl, Decyl, Isodecyl, Undecyl, Dodecyl, Tridecyl, Isotridecyl, Tetradecyl, Pentadecyl, Hexadecyl, Heptadecyl, Octadecyl, Nonadecyl, Eicosyl (Die obigen Bezeichnungen Isooctyl, Isononyl, Isodecyl und Isotridecyl sind Trivialbezeichnungen und stammen von den nach der Oxosynthese erhaltenen Alkoholen - vgl. dazu Ullmanns Enzyklopädie der technischen Chemie, 4. Auflage, Band 7, Seiten 215 bis 217, sowie Band 11, Seiten 435 und 436.), 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 2- oder 4-Butoxybutyl, 4,8-Dioxadecyl, 3,6,9-Trioxadecyl, 3,6,9-Trioxaundecyl, 3,6,9,12-Tetraoxatridecyl, 3,6,9,12-Tetraoxatetradecyl, 2-Carboxyethyl, 2-Methoxycarbonylethyl, Benzyl, 1- oder 2-Phenylethyl, 3-Benzyloxypropyl, Phenoxymethyl, 6-Phenoxy-4-oxahexyl, 8-Phenoxy-4-oxaoctyl, 2-, 3- oder 4-Methylphenyl, 2-, 3- oder 4-Methoxyphenyl, 2-, 3- oder 4-Chlorphenyl, 2-, 3- oder 4-Nitrophenyl oder 2-, 3- oder 4-Carboxylphenyl.

Reste $R^2$ sind z.B. Aminomethyl, N-Mono- oder N,N-Dimethylaminomethyl, N-Mono- oder N,N-Diethylaminomethyl, N-Mono- oder N,N-Dipropylaminomethyl, Morpholinomethyl, Methoxymethyl, Ethoxymethyl, Propoxymethyl, Isopropoxymethyl oder Butoxymethyl.

Reste $R^3$ sind z.B. Methoxycarbonyl, Ethoxycarbonyl, Propoxycarbonyl, Isopropoxycarbonyl, Butoxycarbonyl, Isobutoxycarbonyl oder sec-Butoxycarbonyl.

Reste $R^4$ sind z.B. Amino, Mono- oder Dimethylamino, Mono- oder Diethylamino, Mono- oder Dipropylamino, Mono- oder Diisopropylamino, Mono- oder Dibutylamino, Pyrrolidino, Piperidino, Morpholino, Piperazino oder N-($C_1$-$C_4$-Alkyl)piperazino.

Bevorzugt sind Triazolopyridine der Formel I, in der $R^3$ Cyano bedeutet.

Eine weitere Aufgabe der vorliegenden Erfindung war es, ein Verfahren bereitzustellen, mittels dessen es gelingen sollte, in Ringposition 2 substituierte 5-Methyl-6-cyano-7-hydroxy[1,2,4]triazolo[1,5-a]pyridine auf einfachem Wege und ohne großen Aufwand herzustellen.

Es wurde nun gefunden, daß die Herstellung der Triazolopyridine der Formel IIa

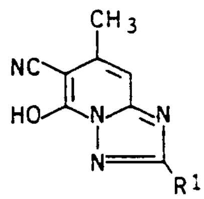

(IIa),

in der

$R^1$ $C_1$-$C_{20}$-Alkyl, das gegebenenfalls durch Phenyl, Phenoxy, Carboxyl oder $C_1$-$C_{20}$-Alkoxycarbonyl, dessen Alkylkette gegebenenfalls durch 1 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alkyl, $C_1$-$C_4$-Alkoxy, Halogen, Nitro oder Carboxyl substituiertes Phenyl oder Hydroxy bedeutet,

vorteilhaft gelingt, wenn man 3-Aminocrotonitril mit einem Hydrazin der Formel III

$A-CO-NH-NH-CO-CH_2CN$ (III),

in der

A Amino oder den obengenannten Rest $R^1$ bedeutet,

gegebenenfalls in Gegenwart eines Dehydratisierungsmittels, bei einer Temperatur von 100 bis 200 °C in einem inerten Lösungsmittel im Molverhältnis 1:1 bis 2:1 umsetzt.

Inerte Losungsmittel sind z.B. Halogenalkane, wie Dichlormethan, Chloroform, Dichlorethan oder Trichlorethan, Halogenbenzole, wie Chlorbenzol, Brombenzol oder o-Dichlorbenzol, höhere Alkanole, wie Pentanol, Hexanol, Heptanol, Octanol oder 2-Ethylhexanol, Ether, wie Ethylenglykolmonomethyl- oder -ethylether, Formamid oder N-Methylpyrrolidon.

Das Molverhältnis von Aminocrotonitril: Hydrazin III beträgt vorzugsweise 1, 25:1.

In manchen Fällen ist es vorteilhaft, die Umsetzung in Gegenwart eines Dehydratisierungsmittels vorzunehmen. Geeignete Dehydratisierungsmittel sind z.B. Carbonsäureanhydride, wie Acetanhydrid, oder Lewis-Säuren, z.B. Zinkchlorid.

Pro Mol Hydrazin III verwendet man im allgemeinen 1 bis 2 Mol an Dehydratisierungsmittel.

Das erfindungsgemäße Verfahren wird üblicherweise bei einer Temperatur von 100 bis 200 °C durchgeführt.

Nach beendeter Umsetzung, die im allgemeinen 2 bis 8 Stunden in Anspruch nimmt, liegt das Zielprodukt IIa üblicherweise als Niederschlag vor und kann abfiltriert und gegebenenfalls weiter gereinigt werden.

Es wurde weiterhin gefunden, daß die Herstellung von Triazolopyridinen der Formel IIb

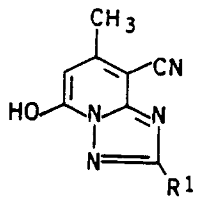

(IIb),

in der

$R^1$ $C_1$-$C_{20}$-Alkyl, das gegebenenfalls durch Phenyl, Phenoxy, Carboxyl oder $C_1$-$C_{20}$-Alkoxycarbonyl, dessen Alkylkette gegebenenfalls durch 1 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alkyl, $C_1$-$C_4$-Alkoxy, Halogen, Nitro oder Carboxyl substituiertes Phenyl oder Hydroxy bedeutet,

vorteilhaft gelingt, wenn man ein Hydrazin der Formel IV

$A\text{-}CO\text{-}NH\text{-}NH\text{-}CO\text{-}CH_2COCH_3$ (IV),

in der

A Amino oder den obengenannten Rest $R^1$ bedeutet,

in basischem Milieu mit Malondinitril bei einer Temperatur von 50 bis 120 °C in einem Alkohol als Lösungsmittel umsetzt und anschließend bei einer Temperatur von 80 bis 120 °C einer Säurebehandlung unterwirft.

Das erfindungsgemäße Verfahren wird in einem Alkohol, z.B. Methanol oder Ethanol, vorgenommen. Als basische Medien dienen z.B. Alkalialkanolate, wie Natriummethanolat oder Natriumethanolat.

Die Umsetzung findet in der Regel bei einer Temperatur von 50 bis 120 °C statt. Danach wird auf Wasser ausgetragen und neutralisiert.

Vor der Säurebehandlung, die üblicherweise in Essigsaure vorgenommen wird, empfiehlt es sich, das als Niederschlag vorliegende Produkt zu isolieren. Die Säurebehandlung wird üblicherweise bei einer Temperatur von 80 bis 120 °C vorgenommen.

Das Hydrazinderivat der Formel IV ist aus Diketen und dem Hydrazin der Formel XI

$A\text{-}CO\text{-}NH\text{-}NH_2$ (XI),

in der A die obengenannte Bedeutung besitzt, zugänglich.

Die übrigen Triazolopyridine der Formel I können aus den 4- oder 6-Cyano-7-hydroxytriazolopyridinen der Formel IIa oder IIb auf an sich bekanntem Wege hergestellt werden.

Beispielsweise kann der Substituent $R^2$ mittels einer elektrophilen Substitutionsreaktion, z.B. Vilsmeier-Reaktion, oder Nitrosierung, gegebenenfalls mit anschließender Derivatisierung, eingeführt werden.

Die Cyanogruppe in Position 6 läßt sich nach an sich bekannten Methoden in die Carbamoyl-, Carboxyl- oder $C_1$-$C_4$-Alkoxycarbonylgruppe überführen.

Durch Behandlung mit Säurehalogeniden, insbesondere Säurechloriden, z.B. Phosphoroxytrichlorid, kann die Hydroxygruppe in Position 7 durch Halogen ersetzt werden.

Das Halogenatom wiederum läßt sich nach an sich bekannten Methoden durch CH-acide Verbindungen oder Amine der Formel XII

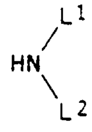

(XII),

in der $L^1$ und $L^2$ jeweils die obengenannte Bedeutung besitzen, austauschen.

Die neuen Triazolopyridine der Formel I sind wertvolle Zwischenprodukte für die Synthese von Farbstoffen und Wirkstoffen. Farbstoffe, die sich von den neuen Triazolopyridinen ableiten, sind z.B. in der

EP-A-416 434 oder DE-A-4 020 768 beschrieben.

Die folgenden Beispiele sollen die Erfindung näher erläutern.

Beispiel 1

(6-Cyano-2,5-dimethyl-7-hydroxy[1,2,4]triazolo[1,5-a]pyridin)

In 250 ml entwässertes Chlorbenzol trug man 120 g 3-Aminocrotonitril ein und rührte bei 110 bis 120 °C. Dann trug man 141 g N-Acetyl-N'-cyanoacetylhydrazin portionsweise ein, wobei man nach jeder Zugabe wartete, bis die exotherme Reaktion abgeklungen war. Das Reaktionsgemisch wurde anschließend 2 Stunden gekocht und nach Zugabe von 125 g Acetanhydrid weitere 2 Stunden unter Rückfluß gehalten. Dann wurde die Heizung abgestellt; bei 60 bis 70 °C gab man 500 ml Methanol zu und ließ auf Raumtemperatur abkühlen. Die resultierende Suspension wurde abgesaugt, der Rückstand mit Methanol gewaschen und bei 80 ° getrocknet. Man erhielt 140 g 6-Cyano-2,5-dimethyl-7-hydroxy[1,2,4]triazolo[1,5-a]-pyridin als farbloses Pulver, das bis 350 ° nicht schmilzt. Die Verbindung löst sich in verdünnten wäßrigen Alkalien und zeigt, nach Umkristallisieren aus N-Methylpyrrolidon, folgende Analysenwerte:

| $C_9H_8N_4O$ (188) | | | | | | | | |
|---|---|---|---|---|---|---|---|---|
| ber:<br>gef: | C | 57,4<br>57,4 | H | 4,3<br>4,4 | N | 29,8<br>29,5 | O | 8,5<br>9,9 |

Beispiel 2

(6-Cyano-2-(1'-ethylpentyl)-7-hydroxy-5-methyl[1,2,4]triazolo[1,5-a]pyridin)

Ein Gemisch aus 750 ml n-Pentanol, 120 g 3-Aminocrotonitril und 225 g N-Cyanoacetyl-N'-(2-ethylhexanoyl)hydrazin wurde 2 Stunden unter Rückfluß gekocht. Nach Zugabe von 150 g wasserfreiem Zinkchlorid wurde weitere 4 Stunden gekocht. Dann gab man 200 ml konz. Salzsäure zu und destillierte das Pentanol mit Wasserdampf ab. Die verbleibende Suspension wurde abgesaugt, der Rückstand mit Wasser gewaschen und unter vermindertem Druck bei 80 °C getrocknet. Man erhielt 265 g 6-Cyano-2-(1'-ethylpentyl)-7-hydroxy-5-methyl[1,2,4]triazolo[1,5-a]pyridin als farblose Pulver. Eine aus Essigsäure umkristallisierte Probe zeigt den Schmelzpunkt 253 bis 254 °C und folgende Analysenwerte:

| $C_{15}H_{20}N_4O$ (272) | | | | | | | | |
|---|---|---|---|---|---|---|---|---|
| ber:<br>gef: | C | 66,2<br>66,0 | H | 7,4<br>7,5 | N | 20,6<br>20,7 | O | 5,9<br>5,9 |

Beispiel 3

(6-Cyano-2,7-dihydroxy-5-methyl[1,2,4]triazolo[1,5-a]pyridin)

In 1000 ml n-Pentanol wurden 115 g 3-Aminocrotonitril und 142 g N-Carbamoyl-N'-cyanoacetylhydrazin eingetragen. Man erhitzte innerhalb einer Stunde zum Sieden und hielt 6 Stunden unter Rückfluß. Nach Erkalten wurde die Suspension abgesaugt und der Rückstand mit Methanol gewaschen. Nach dem Trocknen erhielt man 134 g 6-Cyano-2,7-dihydroxy-5-methyl[1,2,4]triazolo[1,5-a]pyridin als Ammoniumsalz, das bis 350 °C nicht schmilzt. Eine aus Wasser umkristallisierte Probe zeigt folgende Analysenwerte:

| $C_8H_9N_5O_2$ (207) | | | | | | | | |
|---|---|---|---|---|---|---|---|---|
| ber:<br>gef: | C | 46,3<br>46,2 | H | 4,4<br>4,4 | N | 33,8<br>33,4 | O | 15,5<br>15,7 |

Beispiel 4

(6-Cyano-7-hydroxy-5-methyl-2-phenoxymethyl[1,2,4]triazolo[1,5-a]pyridin)

In 250 ml entwässertes o-Dichlorbenzol wurden 125 g 3-Aminocrotonitril eingetragen und bei 100 °C gerührt. Dazu gab man portionsweise 233 g N-Cyanoacetyl-N'-phenoxyacetylhydrazin und rührte anschließend 2 Stunden bei 140 °C. Nach Zugabe von 120 g Acetanhydrid wurde 2 Stunden unter Rückfluß gekocht und während des Erkaltens mit 500 ml Methanol versetzt. Nach dem Erkalten wurde der Niederschlag abgesaugt, mit Methanol nachgewaschen und bei 80 °C unter vermindertem Druck getrocknet. Man erhielt 213 g 6-Cyano-7-hydroxy-5-methyl-2-phenoxymethyl[1,2,4]triazolo[1,5-a]pyridin als farbloses Pulver. Eine aus γ-Butyrolacton umkristallisierte Probe schmilzt nicht bis 350 °C und zeigt folgende Analysewerte:

| $C_{15}H_{12}N_4O_2$ (280) | | | | | | | | |
|---|---|---|---|---|---|---|---|---|
| ber:<br>gef: | C | 64,3<br>64,0 | H | 4,3<br>4,4 | N | 20,0<br>20,0 | O | 11,4<br>11,8 |

Beispiel 5

(2-Benzyl-6-cyano-7-hydroxy-5-methyl[1,2,4]triazolo[1,5-a]pyridin)

120 g 3-Aminocrotonitril wurden unter Erwärmen auf 50 bis 100 °C in 750 ml Ethylenglykolmonoethylether gelöst. Dazu gab man 217 g N-Cyanoacetyl-N'-phenylacetylhydrazin und kochte 8 Stunden unter Rückfluß. Das Reaktionsgemisch wurde nach dem Erkalten auf 1200 ml Wasser und 150 ml konz. Salzsäure gegossen, wobei das Reaktionsprodukt ausfiel. Nach Absaugen, Waschen mit Wasser und Trocknen bei 80 °C unter vermindertem Druck erhielt man 184 g 2-Benzyl-6-cyano-7-hydroxy-5-methyl-[1,2,4]triazolo[1,5-a]pyridin als farbloses Pulver. Eine aus Essigsäure/N,N-Dimethylformamid (3:1 v/v) umkristallisierte Probe zeigt den Schmelzpunkt 319 bis 320 °C und die folgenden Analysenwerte:

| $C_{15}H_{12}N_4O$ (264) | | | | | | | | |
|---|---|---|---|---|---|---|---|---|
| ber:<br>gef: | C | 68,2<br>68,1 | H | 4,6<br>4,6 | N | 21,2<br>21,2 | O | 6,1<br>6,1 |

Beispiel 6

(2-Carboxyethyl-6-cyano-7-hydroxy-5-methyl[1,2,4]triazolo[1,5-a]pyridin)

150 g 3-Aminocrotonitril wurden in 750 ml n-Pentanol gelöst und bei 100 °C gerührt. Dazu gab man in Portionen 199 g N-(3-Carboxypropionyl)-N'-cyanoacetylhydrazin und kochte 2 Stunden unter Rückfluß. Nach Zugabe von 175 g wasserfreiem Zinkchlorid kochte man weitere 4 Stunden und ließ dann 200 ml konz. Salzsäure zulaufen. Nach Abdestillieren des n-Pentanols mit Wasserdampf wurde abgesaugt und mit Wasser gewaschen. Man erhielt nach dem Trocknen bei 80 °C unter vermindertem Druck 121 g 2-Carboxyethyl-6-cyano-7-hydroxy-5-methyl[1,2,4]triazolo[1,5-a]pyridin als farbloses Pulver, das sich in wäßrigem Ammoniak leicht löst. Eine aus γ-Butyrolacton umkristallisierte Probe schmilzt bei 304 bis 305 °C und zeigt folgende Analysewerte:

| $C_{11}H_{10}N_4O_3$ (246) | | | | | | | | |
|---|---|---|---|---|---|---|---|---|
| ber:<br>gef: | C | 53,7<br>53,5 | H | 4,1<br>4,2 | N | 22,7<br>22,4 | O | 19,5<br>19,9 |

Beispiel 7

(6-Cyano-2-ethyl-7-hydroxy-5-methyl[1,2,4]triazolo[1,5-a]pyridin)

In 500 ml Formamid wurden 125 g 3-Aminocrotonitril gelöst und auf 100 °C erwärmt. Dazu gab man 155 g N-Cyanoacetyl-N'-propionylhydrazin in dem Maße, daß nach jeder Zugabe die exotherme Reaktion wieder abklang. Nun rührte man noch 6 Stunden bei 120 bis 125 °C nach und ließ erkalten. Dann verdünnte man mit 1000 ml Wasser und säuerte mit konz. Salzsäure an. Nach Absaugen, Waschen mit Wasser und Trocknen bei 100 °C erhielt man 141 g 6-Cyano-2-ethyl-7-hydroxy-5-methyl[1,2,4]triazolo[1,5-a]pyridin. Eine aus N-Methylpyrrolidon umkristallisierte Probe schmilzt bei 346 bis 348 °C und zeigt folgende Analyse:

| $C_{10}H_{10}N_4O$ (202) | | | | | | | | |
|---|---|---|---|---|---|---|---|---|
| ber:<br>gef: | C | 59,4<br>59,2 | H | 5,0<br>5,0 | N | 27,7<br>27,8 | O | 7,9<br>8,3 |

Beispiel 8

(6-Cyano-2-heptadecyl-7-hydroxy-5-methyl[1,2,4]triazolo[1,5-a]pyridin)

Ein Gemisch aus 1000 ml Ethylenglykolmonoethylether, 120 g 3-Aminocrotonitril und 365 g N-Cyanoacetyl-N'-stearoylhydrazin wurde innerhalb von 2 Stunden auf 135 °C erhitzt und dann weitere 2 Stunden unter Rückfluß gekocht. Nach Zugabe von 150 g wasserfreiem Zinkchlorid kochte mam noch 4 Stunden, verdünnte dann die Schmelze mit 2000 ml Wasser und setzte 200 ml 12 gew.%ige Salzsäure zu. Das ausgefallene Produkt wurde abgesaugt, mit Wasser nachgewaschen und bei 80 °C unter vermindertem Druck getrocknet. Man erhielt 397 g 6-Cyano-2-heptadecyl-7-hydroxy-5-methyl-[1,2,4]triazolo[1,5-a]pyridin. Die Verbindung schmilzt nach Umkristallisieren aus Essigsäure bei 274 bis 275 °C und zeigt folgende Analysenwerte:

| $C_{25}H_{40}N_4O$ (412) | | | | | | | | |
|---|---|---|---|---|---|---|---|---|
| ber:<br>gef: | C | 72,8<br>72,5 | H | 9,8<br>9,8 | N | 13,6<br>13,5 | O | 3,9<br>4,2 |

Beispiel 9

(6-Cyano-7-hydroxy-5-methyl-2-(p-nitrophenyl)[1,2,4]triazolo[1,5-a]pyridin)

Ein Gemisch aus 2000 ml n-Pentanol, 125 g 3-Aminocrotonitril und 248 g N-Cyanoacetyl-N'-(p-nitrobenzoyl)hydrazin wurde 2 Stunden unter Rückfluß gekocht und nach Zugabe von 150 g wasserfreiem Zinkchlorid weitere 2 Stunden gekocht. Dann gab man 150 ml konz. Salzsäure zu und destillierte das n-Pentanol mit wasserdampf ab. Die resultierende Suspension wurde warm abgesaugt, der Rückstand mit Wasser gewaschen und bei 100 °C getrocknet. Man erhielt 256 g 6-Cyano-7-hydroxy-5-methyl-2-(p-nitrophenyl)[1,2,4]triazolo[1,5-a]pyridin. Die Verbindung schmilzt nicht bis 350 °C und zeigt folgende Analysenwerte (aus N-Methylpyrrolidon umkristallisiert):

| $C_{14}H_9N_5O_3$ (295) | | | | | | | | |
|---|---|---|---|---|---|---|---|---|
| ber:<br>gef: | C | 56,9<br>57,9 | H | 3,1<br>3,3 | N | 23,7<br>23,4 | O | 16,3<br>16,2 |

Beispiel 10

Analog Beispiel 9 wurde 6-Cyano-7-hydroxy-5-methyl-2-(m-nitrophenyl)[1,2,4]triazolo[1,5-a]pyridin erhalten, das ebenfalls nicht bis 350° schmilzt.

Beispiel 11

(6-Cyano-2-(1'-ethylpentyl)-4-formyl-7-hydroxy-5-methyl[1,2,4]triazolo[1,5-a]pyridin)

In 1000 ml Chloroform wurden 120 g N,N-Dimethylformamid und 272 g 6-Cyano-2-(1'-ethylpentyl)-7-hydroxy-5-methyl[1,2,4]triazolo[1,5-a]pyridin eingetragen und bei Raumtemperatur gerührt. Dazu tropfte man 250 g Phosphoroxytrichlorid und kochte anschließend 6 Stunden unter Rückfluß. Dann setzte man 250 ml Wasser zu und destillierte das Chloroform mit Wasserdampf ab. Die verbleibende Suspension wurde abgesaugt, der Rückstand mit Wasser gewaschen und unter vermindertem Druck bei 80°C getrocknet. Man erhielt 265 g der Verbindung der Formel

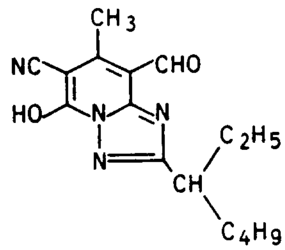

vom Schmelzpunkt 214 bis 215°C (aus Ethanol).
Analyse:

| $C_{16}H_{20}N_4O_2$ (300) | | | | | | | | |
|---|---|---|---|---|---|---|---|---|
| ber:<br>gef: | C | 64,0<br>64,0 | H | 6,7<br>6,9 | N | 18,6<br>18,6 | O | 10,7<br>10,8 |

Analog Beispiel 11 wurden die folgenden Verbindungen der Formel

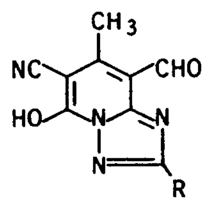

erhalten.

| Bsp.-Nr. | R | Fp. (umkristallisiert aus) |
|---|---|---|
| 12 | $CH_3$ | 336 - 337°C (N-Methylpyrrolidon) |
| 13 | $(CH_2)_{16}CH_3$ | 205 - 206°C (n-Pentanol) |

Beispiel 14

(7-Chlor-6-cyano-2-(1'-ethylpentyl)-5-methyl[1,2,4]triazolo[1,5-a]pyridin)

In 250 g Phosphoroxytricfllorid wurden 272 g 6-Cyano-2-(1'-ethylpentyl)-7-hydroxy-5-methyl[1,2,4]-triazolo[1,5-a]pyridin eingetragen und 4 Stunden bei 105 °C gerührt. Dann tropfte man 250 g Methanol zu, kochte kurz auf und ließ 250 ml Wasser zulaufen. Die Lösung wurde gekühlt und bei 10 bis 20 °C mit wäßriger Ammoniaklösung auf pH 7,5 gestellt, wobei das Zielprodukt ausfiel. Es wurde abgesaugt, mit Wasser gewaschen und unter vermindertem Druck bis 50 °C getrocknet. Man erhielt 264 g der Verbindung mit der Formel

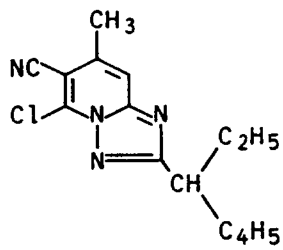

Eine aus Ethanol/Cyclohexan umkristallisierte Probe weist einen Schmelzpunkt von 95 bis 96 °C auf und ergibt folgende Analyse:

| $C_{15}H_{19}ClN_4$ (209,5) | | | | | | | | |
|---|---|---|---|---|---|---|---|---|
| ber:<br>gef: | C | 62,0<br>62,0 | H | 6,6<br>6,7 | N | 12,2<br>12,0 | O | 19,3<br>19,2 |

Analog Beispiel 14 wurden die folgenden Verbindungen der Formel

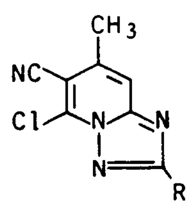

erhalten.

| Bsp.-Nr. | R | Fp. (umkristallisiert aus) | Cl ber. | Cl gef. |
|---|---|---|---|---|
| 15 | $CH_3$ | 202 - 203 °C (Essigsäure) | 17,2 | 16,9 % |
| 16 | $C_2H_5$ | 162 - 163 °C (Ethanol) | 16,8 | 16,5 % |
| 17 | $C_6H_5CH_2$ | 177 - 178 °C (n-Pentanol) | 12,5 | 12,2 % |
| 18 | $C_6H_5OCH_2$ | 187 - 188 °C (Essigsäure) | 11,9 | 11,8 % |
| 19 | $C_{17}H_{19}$ | 110 °C (Ethanol) | 8,3 | 8,2 % |

Beispiel 20

(2-Benzyl-6-cyano-7-dimethylamino-5-methyl[1,2,4]triazolo[1,5-a]pyridin)

282,5 g der nach Beispiel 17 erhaltenen Verbindung wurden in 1000 ml Ethanol zum Sieden erhitzt. Dazu tropfte man 300 g 40 gew.%ige wäßrige Dimethylaminlösung innerhalb einer Stunde und kochte

anschließend 6 Stunden unter Rückfluß. Dabei ging das Ausgangsprodukt vollständig in Lösung, während des Erkaltens kristallisierte das Endprodukt aus. Man erhielt nach dem Absaugen und Trocknen 270 g der Verbindung der Formel

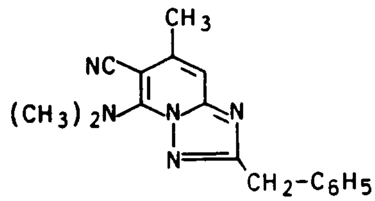

mit einem Schmelzpunkt von 130 bis 131 °C (aus Ethanol).

Analyse:

| $C_{17}H_{17}N_5$ (291) | | | | | | |
|---|---|---|---|---|---|---|
| ber:<br>gef: | C | 70,1<br>70,0 | H | 5,9<br>6,0 | N | 24,0<br>24,2 |

Analog Beispiel 20 wurden die folgenden Verbindungen der Formel

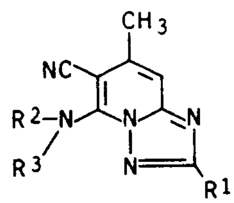

erhalten.

| Bsp.-Nr. | R1 | R2 | R3 | Fp. (umkristallisiert aus) | | C | H | N | O |
|---|---|---|---|---|---|---|---|---|---|
| 21 | $CH_3$ | $CH_3$ | $CH_3$ | 153 - 154°C (Ethanol) | ber: | 61,4 | 6,1 | 32,5 | - |
| | | | | | gef: | 61,5 | 6,2 | 32,4 | - |
| 22 | $CH_3$ | $C_2H_5$ | $C_2H_5$ | 86 - 87°C (Cyclohexan/Ethanol) | ber: | 64,2 | 7,0 | 28,8 | - |
| | | | | | gef: | 64,4 | 7,0 | 28,8 | - |
| 23 | 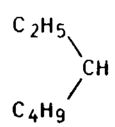 | $(CH_2)_2O(CH_2)_2$ | | 90 - 91°C (Methanol) | ber: | 66,8 | 8,0 | 20,5 | 4,7 |
| | | | | | gef: | 67,1 | 8,2 | 20,4 | 4,8 |
| 24 | $C_6H_5CH_2$ | $CH_3$ | $CH_2CH_2OH$ | 119 - 120°C (Ethanol) | ber: | 67,3 | 6,0 | 21,8 | 5,0 |
| | | | | | gef: | 67,3 | 6,1 | 21,7 | 5,2 |
| 25 | $C_6H_5CH_2$ | $(CH_2)_2O(CH_2)_2$ | | 131 - 132°C (Ethanol) | ber: | 68,5 | 5,7 | 21,0 | 4,8 |
| | | | | | gef: | 68,5 | 6,0 | 20,9 | 5,1 |

Beispiel 26

14,5 g der in Beispiel 14 beschriebenen Verbindung wurden mit 9,9 g Malondinitril und 15,3 g Triethylamin in 300 ml N,N-Dimethylformamid 5 Stunden auf 90 °C erwärmt. Dann wurde auf 1,5 l Wasser gegossen, mit konz. Salzsäure auf pH 5 angesäuert, der Niederschlag abgesaugt, mit Wasser gewaschen und bei 50 °C unter vermindertem Druck getrocknet. Man erhielt 13,5 g der Verbindung der

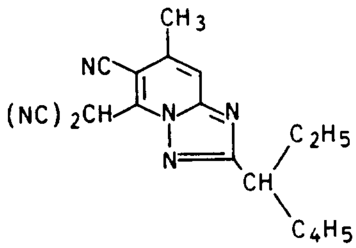

Eine aus Ethanol umkristallisierte Probe zeigt einen Schmelzpunkt von 231 bis 233 °C und ergibt folgende Analysenwerte:

| $C_{18}H_{20}N_6$ (320,4) | | | | | | |
|---|---|---|---|---|---|---|
| ber:<br>gef: | C | 67,5<br>67,1 | H | 6,3<br>6,4 | N | 26,2<br>26,2 |

Analog Beispiel 26 wurden die folgenden Verbindungen der Formel

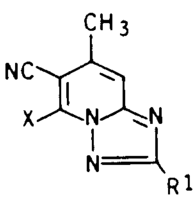

erhalten.

| Bsp.-Nr. | $R^1$ | X | Fp. (umkrist. aus) | Analysenwerte |
|---|---|---|---|---|
| 27 | $(CH_2)_{16}CH_3$ | $CH(CN)_2$ | 245 -248°C (Ethanol) | $C_{28}H_{40}N_6$ (460,7)<br>ber: C 73,0 H 8,8 N 18,2<br>gef: 72,3 8,8 18,1 |
| 28 | $(CH_2)_{16}CH_3$ | 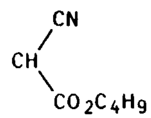 | 97 - 98°C (Ethanol) | $C_{32}H_{49}N_5O_2$ (535,8)<br>ber: C 71,7 H 9,2 N 13,1 O 6,0<br>gef: 71,5 9,4 12,7 6,1 |
| 29 | $CH(C_2H_5)C_4H_9$ | 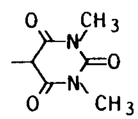 | 122 - 124°C (Ethanol/$H_2O$) | $C_{21}H_{30}N_6O_5 \cdot 2H_2O$ (446,5)<br>ber: C 56,5 H 6,8 N 18,8 O 17,9<br>gef: 56,7 7,1 18,4 17,9 |

Beispiel 30

2,72 g der in Beispiel 2 beschriebenen Verbindung wurden in 30 ml 60 gew.%iger Schwefelsäure 10 Stunden auf 120 ° C erhitzt. Dann wurde die Mischung auf 300 ml Eiswasser gegossen, mit 50 gew.%iger Natronlauge neutralisiert, der Niederschlag abgesaugt, mit Wasser gewaschen und bei 50 ° C unter vermindertem Druck getrocknet. Man erhielt 1,8 g der Verbindung der Formel

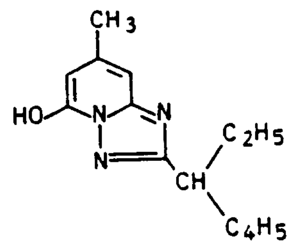

Eine aus Ethanol umkristallisierte Probe zeigt einen Schmelzpunkt von 194 ° C und ergibt folgende Analysenwerte :

| $C_{14}H_{21}N_3O$ (247,3) | | | | | | | | |
|---|---|---|---|---|---|---|---|---|
| ber:<br>gef: | C | 68,0<br>67,6 | H | 8,6<br>8,7 | N | 17,0<br>16,9 | O | 6,5<br>6,7 |

Beispiel 31

(6-Cyano-2-(1'-ethylpentyl)-7-hydroxy-5-methyl-4-nitroso[1,2,4]triazolo[1,5-a]pyridin)

5,44 g der in Beispiel 2 beschriebenen Verbindung wurden in 30 ml Eisessig und 30 ml konz. Salzsäure gelöst. Bei 0 bis 5 ° C wurden 1,45 g Natriumnitrit, gelöst in 5 ml Wasser, zugetropft. Bei dieser Temperatur wurde 2 Stunden gerührt. Dann wurde auf 300 ml Wasser gegossen, 30 Minuten gerührt, der Niederschlag abgesaugt und getrocknet. Man erhielt 3,7 g 6-Cyano-2-(1'-ethylpentyl)-7-hydroxy-5-methyl-4-nitroso[1,2,4]-triazolo[1,5-a]pyridin als grünes Pulver. Eine aus Toluol umkristallisierte Probe schmilzt bei 129 bis 130 ° C und zeigt folgende Analysenwerte:

| $C_{15}H_{19}N_5O_2$ (301,4) | | | | | | | | |
|---|---|---|---|---|---|---|---|---|
| ber:<br>gef: | C | 59,8<br>59,3 | H | 6,4<br>6,5 | N | 23,2<br>23,0 | O | 10,6<br>10,8 |

Das $^1$H-NMR-Spektrum steht mit der Konstitution im Einklang.

Beispiel 32

(4-Cyano-7-hydroxy-5-methyl-2-phenyl[1,2,4]triazolo[1,5-a]pyridin

Zu einem Gemisch aus 600 ml Methanol, 66 g Malondinitril und 220 g N-Benzoyl-N'-acetoacetylhydrazin wurden 180 g 30 gew.-%ige methanolische Natriummethylatlösung getropft. Nach 6-stündigem Erhitzen unter Rückfluß wurde das Reaktionsgemisch auf Eiswasser ausgetragen und durch Zugabe von 60 g Essigsäure neutral gestellt. Das ausgefallene farblose Produkt wurde abgesaugt, mit Wasser gewaschen und getrocknet. Ausbeute: 248 g. Dann wurde das Produkt in 1000 ml Essigsäure eingetragen und 4 Stunden unter Rühren und Rückfluß erhitzt. Nach Erkalten wurde mit dem gleichen Volumen Wasser verdünnt, abgesaugt, mit Wasser gewaschen und getrocknet. Man erhielt 192 g der Verbindung der Formel

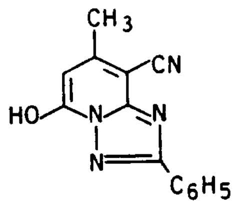

Eine Probe wurde aus N,N-Dimethylformamid/Essigsäure (1:1 v/v) umkristallisiert und zeigt einen Schmelzpunkt von 342 bis 343 ° C.

| Analyse: $C_{14}H_{10}N_4O$ (250) | | | | | | | | |
|---|---|---|---|---|---|---|---|---|
| ber:<br>gef: | C | 67,1<br>66,9 | H | 4,0<br>4,0 | N | 22,4<br>22,2 | O | 6,4<br>6,7 |

Beispiel 33

(4-Cyano-7-chlor-5-methyl-2-phenyl[1,2,4-triazolo[1,5-a]pyridin)

In 500 ml Phosphoroxytrichlorid wurden 250 g der nach Beispiel 32 erhaltenen Verbindung eingetragen und 6 Stunden unter Rückfluß gekocht. Das Reaktionsgemisch wurde auf Eiswasser ausgetragen, das ausgefallene Produkt abgesaugt und mit Wasser gewaschen. Nach dem Trocknen wurden 265 g der farblosen Verbindung der Formel

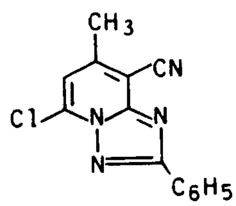

erhalten, die nach Umkristallisieren aus Essigsäure einen Schmelzpunkt von 223 bis 224 ° C zeigt.
Chlor ber.: 13,2, gef.: 13,1.

## Patentansprüche

**1.** Triazolopyridine der Formel I

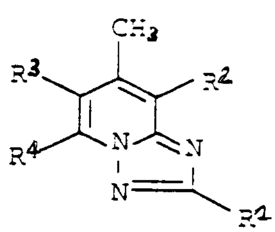

(I),

in der

$R^1$ $C_1$-$C_{20}$-Alkyl, das gegebenenfalls durch Phenyl, Phenoxy, Carboxyl oder $C_1$-$C_{20}$-Alkoxycarbonyl, dessen Alkylkette gegebenenfalls durch 1 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alyl, $C_1$-$C_4$-Alkoxy, Halogen, Nitro oder Carboxyl substituiertes Phenyl oder Hydroxy,

$R^2$ Wasserstoff, Formyl, Nitroso, Cyano, $C_1$-$C_4$-Alkoxymethyl oder einen Rest der Formel $CH_2$-

$NL^1L^2$, wobei $L^1$ und $L^2$ gleich oder verschieden sind und unabhängig voneinander jeweils Wasserstoff oder zusammen mit dem sie verbindenden Stickstoffatom einen 5- oder 6-gliedrigen gesättigten heterocyclischen Rest,

$R^3$ Wasserstoff, Cyano, Carbamoyl, Carboxyl oder $C_1$-$C_4$-Alkoxycarbonyl und

$R^4$ Hydroxy, Halogen, den Rest $-NL^1L^2$, wobei $L^1$ und $L^2$ jeweils die obengenannte Bedeutung besitzen, oder den Rest einer CH-aciden Verbindung bedeuten, die sich von Nitromethan, Nitroethan oder von Verbindungen der Formeln V bis X

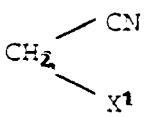
(V)

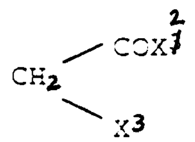
(VI)

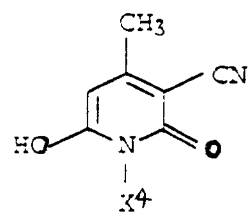
(VII)

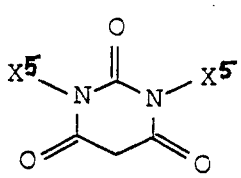
(VIII)

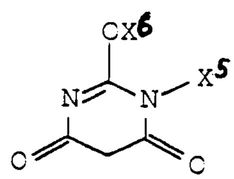
(IX)

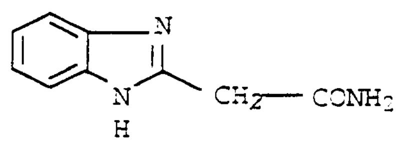
(X)

ableitet, wobei

$X^1$ Cyano, Nitro, $C_1$-$C_4$-Alkanoyl, gegebenenfalls durch $C_1$-$C_4$-Alkyl, $C_1$-$C_4$-Alkoxy oder Halogen substituiertes Benzoyl, $C_1$-$C_6$-Alkylsulfonyl, gegebenenfalls durch $C_1$-$C_4$-Alkyl, $C_1$-$C_4$-Alkoxy oder Halogen substituiertes Phenylsulfonyl, Carboxyl, $C_1$-$C_4$-Alkoxycarbonyl, Phenoxycarbonyl, Carbamoyl, $C_1$-$C_4$-Mono- oder Dialkylcarbamoyl, gegebenenfalls durch $C_1$-$C_4$-Alkyl, $C_1$-$C_4$-Alkoxy oder Halogen substituiertes Phenylcarbamoyl, gegebenenfalls durch $C_1$-$C_4$-Alkyl, $C_1$-$C_4$-Alkoxy, Halogen oder Nitro substituiertes Phenyl, Benzthiazol-2-yl, Benzimidazol-2-yl, 5-Phenyl-1,3,4-thiadiazol-2-yl oder 2-Hydroychinoxalin-3-yl,

$X^2$ $C_1$-$C_4$-Alkyl oder $C_1$-$C_4$-Alkoxy,

$X^3$ $C_1$-$C_4$-Alkoxycarbonyl, Phenylcarbamoyl oder Benzimidazol-2-yl,

$X^4$ Wasserstoff oder $C_1$-$C_6$-Alkyl,

$X^5$ Wasserstoff, $C_1$-$C_4$-Alkyl oder Phenyl und

$X^5$ $C_1$-$C_4$-Alkyl bedeuten.

**2.** Triazolopyridine gemäß Anspruch 1, dadurch gekennzeichnet, daß $R^3$ Cyano bedeutet.

**3.** Verfahren zur Herstellung von Triazolopyridinen der Formel IIa

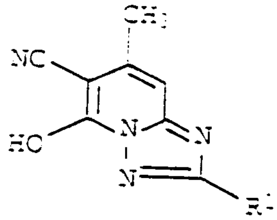
(IIa),

in der

$R^1$ $C_1$-$C_{20}$-Alkyl, das gegebenenfalls durch Phenyl, Phenoxy, Carboxyl oder $C_1$-$C_{20}$-Alkoxycarbonyl, dessen Alkylkette gegebenenfalls durch 1 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alkyl, $C_1$-$C_4$-Alkoxy, Halogen, Nitro oder Carboxyl substituiertes Phenyl oder Hydroxy bedeutet,

dadurch gekennzeichnet, daß man 3-Aminocrotonitril mit einem Hydrazin der Formel III

A-CO-NH-NH-CO-$CH_2$CN (III),

in der

A Amino oder den obengenannten Rest $R^1$ bedeutet,

gegebenefalls in Gegenwart eines Dehydratisierungsmittels, bei einer Temperatur von 100 bis 200 °C in einem inerten Lösungsmittel im Molverhältnis 1:1 bis 2:1 umsetzt.

4. Verfahren zur Herstellung von Triazolopyridinen der Formel IIb

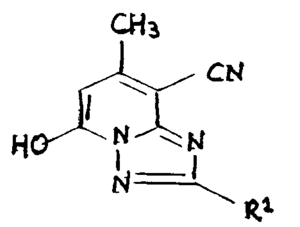

(IIb),

in der

$R^1$ $C_1$-$C_{20}$-Alkyl, das gegebenenfalls durch Phenyl, Phenoxy, Carboxyl oder $C_1$-$C_{20}$-Alkoxycarbonyl, dessen Alkylkette gegebenefalls durch 2 bis 4 Sauerstoffatome unterbrochen und/oder durch Phenyl oder Phenoxy substituiert ist, und durch 1 bis 4 Sauerstoffatome unterbrochen sein kann, gegebenenfalls durch $C_1$-$C_4$-Alkyl, $C_1$-$C_4$-Alkoxy, Halogen, Nitro oder Carboxyl substituiertes Phenyl oder Hydroxy bedeutet,

dadurch gekennzeichnet, daß man ein Hydrazin der Formel IV

A-CO-NH-NH-CO-$CH_2$$COCH_3$ (IV),

in der

A Amino oder den obergennanten Rest $R^1$ bedeutet,

in basischem Milieu mit Malondinitril bei einer Temperatur von 50 bis 120 °C in einem Alkohol als Lösungsmittel umsetzt und anschließend bei einer Temperatur von 80 bis 120 °C einer Säurebehandlung unterwirft.

## Claims

1. A triazolopyridine of the formula I

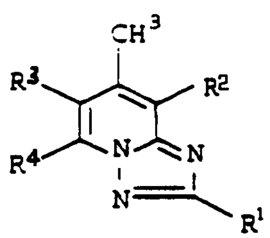

(I),

where

$R^1$ is $C_1$-$C_{20}$-alkyl which is unsubstituted or substituted by phenyl, phenoxy, carboxyl or $C_1$-$C_{20}$-

alkoxycarbonyl whose alkyl chain may be interrupted by 1 to 4 oxygen atoms and/or substituted by phenyl or phenoxy, and may be interrupted by 1 to 4 oxygen atoms, unsubstituted or $C_1$-$C_4$-alkyl, $C_1$-$C_4$-alkoxy, halogen, nitro or carboxyl substituted phenyl or hydroxyl,

$R^2$ is hydrogen, formyl, nitroso, cyano, $C_1$-$C_4$-alkoxymethyl or $CH_2$-$NL^1L^2$ where $L^1$ and $L^2$ are identical or different and each, independently of one another, is hydrogen, or they form together with the nitrogen connecting them a 5- or 6-membered saturated heterocyclic radical,

$R^3$ is hydrogen, cyano, carbamoyl, carboxyl or $C_1$-$C_4$-alkoxycarbonyl and

$R^4$ is hydroxyl, halogen, where -$NL^1L^2$ $L^1$ and $L^2$ each have the abovementioned meaning, or the radical of a compound with an acidic CH, which is derived from nitromethane, nitroethane or from compounds of the formulae V to X

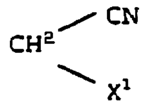

(V)

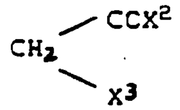

(VI)

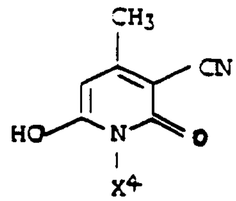

(VII)

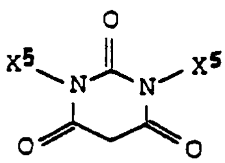

(VIII)

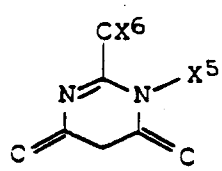

(IX)

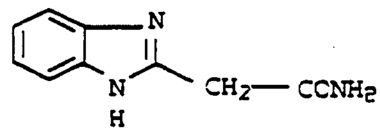

(X)

where

$X^1$ is cyano, nitro, $C_1$-$C_4$-alkanoyl, unsubstituted or $C_1$-$C_4$-alkyl, $C_1$-$C_4$-alkoxy or halogen substituted benzoyl, $C_1$-$C_6$-alkylsulfonyl, unsubstituted or $C_1$-$C_4$-alkyl, $C_1$-$C_4$-alkoxy or halogen substituted phenylsulfonyl, carboxyl, $C_1$-$C_4$-alkoxycarbonyl, phenoxycarbonyl, carbamoyl, $C_1$-$C_4$-mono- or dialkylcarbamoyl, unsubstituted or $C_1$-$C_4$-alkyl, $C_1$-$C_4$-alkoxy or halogen substituted phenylcarbamoyl, unsubstituted or $C_1$-$C_4$-alkyl, $C_1$-$C_4$-alkoxy, halogen or nitro substituted phenyl, benzothiazol-2-yl, benzimidazol-2-yl, 5-phenyl-1,3,4-thiadiazol-2-yl or 2-hydroxyquinoxalin-3-yl,

$X^2$ is $C_1$-$C_4$-alkyl or $C_1$-$C_4$-alkoxy,

$X^3$ is $C_1$-$C_4$-alkoxycarbonyl, phenylcarbamoyl or benzimidazol-2-yl,

$X^4$ is hydrogen or $C_1$-$C_6$-alkyl,

$X^5$ is hydrogen, $C_1$-$C_4$-alkyl or phenyl and

$X^5$ is $C_1$-$C_4$-alkyl.

**2.** A triazolopyridine as claimed in claim 1, wherein $R^3$ is cyano.

**3.** A process for the preparation of a triazolopyridine of the formula IIa

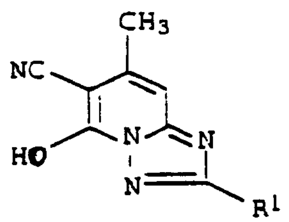

(IIa),

where

$R^1$ is $C_1$-$C_{20}$-alkyl which is unsubstituted or substituted by phenyl, phenoxy, carboxyl or $C_1$-$C_{20}$-alkoxycarbonyl whose alkyl chain may be interrupted by 1 to 4 oxygen atoms and/or substituted by phenyl or phenoxy, and may be interrupted by 1 to 4 oxygen atoms, unsubstituted or $C_1$-$C_4$-alkyl, $C_1$-$C_4$-alkoxy, halogen, nitro or carboxyl substituted phenyl or hydroxyl,

which comprises reacting 3-aminocrotonitrile with a hydrazine of the formula III

A-CO-NH-NH-CO-$CH_2$CN (III)

where

A is amino or the abovementioned $R^1$,

in the presence or absence of a dehydrating agent at from 100 to 200 °C in an inert solvent in the molar ratio from 1:1 to 2:1.

**4.** A process for the preparation of a triazolopyridine of the formula IIb

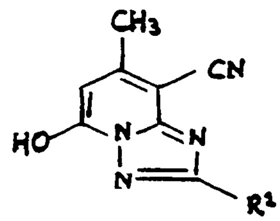

(IIb),

where

$R^1$ is $C_1$-$C_{20}$-alkyl which is unsubstituted or substituted by phenyl, phenoxy, carboxyl or $C_1$-$C_{20}$-alkoxycarbonyl whose alkyl chain may be interrupted by 2 to 4 oxygen atoms and/or substituted by phenyl or phenoxy, and may be interrupted by 1 to 4 oxygen atoms, unsubstituted or $C_1$-$C_4$-alkyl, $C_1$-$C_4$-alkoxy, halogen, nitro or carboxyl substituted phenyl or hydroxyl,

which comprises reacting a hydrazine of the formula IV

A-CO-NH-NH-CO-$CH_2COCH_3$ (IV)

where

A is amino or the abovementioned $R^1$,

in a basic medium with malononitrile at from 50 to 120 °C in an alcohol as solvent and subsequently treating with acid at from 80 to 120 °C.

## Revendications

**1.** Triazolopyridines de formule I

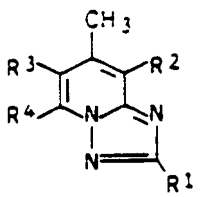

(I),

dans laquelle

$R^1$ représente un reste alkyle en $C_1$-$C_{20}$ qui est éventuellement substitué par un groupement phényle. phénoxy, carboxyle ou (alcoxy en $C_1$-$C_{20}$)carbonyle (dont la chaîne alkyle est eventuellement interrompue par 1 à 4 atomes d'oxygène et/ou substituée par un radical phényle ou phénoxy), et qui peut être interrompu par 1 à 4 atomes d'oxygène, un reste phényle éventuellement substitué par un radical alkyle en $C_1$-$C_4$, alcoxy en $C_1$-$C_4$, par un atome d'halogène ou par un groupement nitro ou carboxyle, ou un reste hydroxy,

$R^2$ représente un atome d'hydrogène, un reste formyle, nitroso, cyano, (alcoxy en $C_1$-$C_4$)méthyle ou un reste de formule $CH_2$-$NL^1L^2$, $L^1$ et $L^2$ étant identiques ou différents et représentant chacun, indépendamment l'un de l'autre, un atome d'hydrogène ou formant ensemble, avec l'atome d'azote qui les relie, un reste hétérocyclique saturé a 5 ou 6 maillons,

$R^3$ représente un atome d'hydrogène ou un groupement cyano, carbamoyle, carboxyle ou (alcoxy en $C_1$-$C_4$)carbonyle et

$R^4$ représente un reste hydroxy, un atome d'halogène, le reste -$NL^1L^2$, $L^1$ et $L^2$ ayant chacun la signification susmentionnée, ou le reste d'un composé a CH acide qui dérive de nitrométhane, de nitroéthane ou de composés de formules V à X

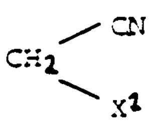

(V)

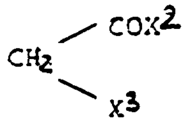

(VI)

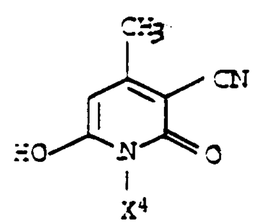

(VII)

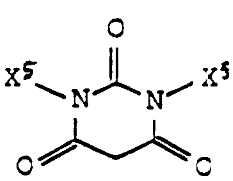

(VIII)

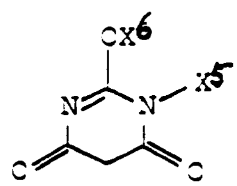

(IX)

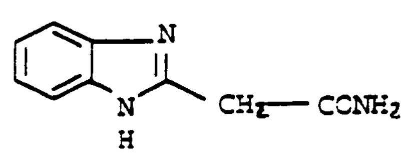

(X)

où

$X^1$ représente un groupement cyano, nitro, alcanoyle en $C_1$-$C_4$, benzoyle éventuellement substitué par un reste alkyle en $C_1$-$C_4$, alcoxy en $C_1$-$C_4$ ou par un atome d'halogène, alkylsulfonyle en $C_1$-$C_6$, phénylsulfonyle éventuellement substitué par un reste alkyle en $C_1$-$C_4$, alcoxy en $C_1$-$C_4$ ou par un atome d'halogène, carboxyle, (alcoxy en $C_1$-$C_4$)carbonyle, phénoxycarbonyle, carbamoyle, mono- ou di(alkyl en $C_1$-$C_4$)carbamoyle, phénylcarbamoyle éventuellement

substitué par un reste alkyle en $C_1$-$C_4$, alcoxy en $C_1$-$C_4$ ou par un atome d'halogène, phényle éventuellement substitué par un reste alkyle en $C_1$-$C_4$, alcoxy en $C_1$-$C_4$, par un atome d'halogène ou par un groupement nitro, benzothiazole-2-yle, benzimidazole-2-yle, 5-phényl-1,3,4-thiadiazole-2-yle ou 2-hydroxyquinoxaline-3-yle,

$X^2$ représente un reste alkyle en $C_1$-$C_4$ ou alcoxy en $C_1$-$C_4$,

$X^3$ représente un groupement (alcoxy en $C_1$-$C_4$)carbonyle, phénylcarbamoyle ou benzimidazole-2-yle,

$X^4$ représente un atome d'hydrogène ou un reste alkyle en $C_1$-$C_6$,

$X^5$ représente un atome d'hydrogène ou un reste alkyle en $C_1$-$C_4$ ou phényle, et

$X^5$ représente un reste alkyle en $C_1$-$C_4$.

**2.** Triazolopyridines selon la revendication 1, caractérisées en ce que $R^3$ représente un groupement cyano.

**3.** Procédé de préparation de triazolopyridines de formule IIa

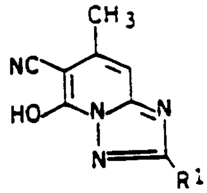 (IIa),

dans laquelle

$R^1$ représente un reste alkyle en $C_1$-$C_{20}$ qui est éventuellement substitué par un groupement phényle, phénoxy, carboxyle ou (alcoxy en $C_1$-$C_{20}$)carbonyle (dont la chaîne alkyle est éventuellement interrompue par 1 à 4 atomes d'oxygène et/ou substituée par un radical phényle ou phénoxy), et qui peut être interrompu par 1 à 4 atomes d'oxygène, un reste phényle éventuellement substitué par un radical alkyle en $C_1$-$C_4$, alcoxy en $C_1$-$C_4$, par un atome d'halogène ou par un groupement nitro ou carboxyle, ou un reste hydroxy,

caractérisé en ce qu'on fait réagir du 3-aminocrotonitrile avec une hydrazine de formule III

$A\text{-}CO\text{-}NH\text{-}NH\text{-}CO\text{-}CH_2CN$ (III)

dans laquelle

A représente un groupement amino ou le reste $R^1$ susmentionné,

dans un rapport molaire de 1:1 à 2:1, éventuellement en présence d'un agent de déshydratation, a une température de 100 à 200 °C, dans un solvant inerte.

**4.** Procédé de préparation de triazolopyridines de formule IIb

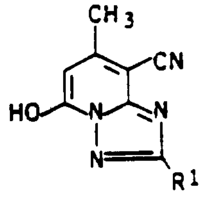 (IIb),

dans laquelle

$R^1$ représente un reste alkyle en $C_1$-$C_{20}$ qui est éventuellement substitué par un groupement phényle, phénoxy, carboxyle ou (alcoxy en $C_1$-$C_{20}$)carbonyle (dont la chaîne alkyle est éventuellement interrompue par 1 à 4 atomes d'oxygène et/ou substituée par un radical phényle ou phénoxy), et qui peut être interrompu par 1 à 4 atomes d'oxygène, un reste phényle éventuellement substitué par un radical alkyle en $C_1$-$C_4$, alcoxy en $C_1$-$C_4$, par un

atome d'halogène ou par un groupement nitro ou carboxyle, ou un reste hydroxy,
caractérisé en ce qu'on fait réagir une hydrazine de formule IV

$A-CO-NH-NH-CO-CH_2COCH_3$ (IV)

dans laquelle
A représente un groupement amino ou le reste $R^1$ susmentionné,
en milieu basique, avec du malodinitrile, à une température de 50 à 120°C, dans un alcool servant de solvant, puis on soumet le produit à un traitement par acide à une température de 80 à 120°C.